(19) Europäisches Patentamt / European Patent Office / Office européen des brevets

(11) **EP 4 032 898 A1**

(12) **EUROPEAN PATENT APPLICATION**
published in accordance with Art. 153(4) EPC

(43) Date of publication:
**27.07.2022 Bulletin 2022/30**

(21) Application number: **20876464.7**

(22) Date of filing: **14.09.2020**

(51) International Patent Classification (IPC):
***C07H 19/20*** (2006.01)   ***C07H 1/08*** (2006.01)

(86) International application number:
**PCT/KR2020/012364**

(87) International publication number:
**WO 2021/075734 (22.04.2021 Gazette 2021/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.10.2019 KR 20190129324**

(71) Applicant: **CJ Cheiljedang Corporation**
**Seoul 04560 (KR)**

(72) Inventors:
• **CHOI, Jung Hwa**
**Seoul 04560 (KR)**
• **KIM, Min Jong**
**Seoul 04560 (KR)**

• **OH, Chang Yub**
**Seoul 04560 (KR)**
• **LIM, Hwa Yeon**
**Seoul 04560 (KR)**
• **KIM, Jun Woo**
**Seoul 04560 (KR)**
• **YU, Jae Hun**
**Seoul 04560 (KR)**
• **KANG, Seok Hyun**
**Seoul 04560 (KR)**
• **KIM, Yu Shin**
**Seoul 04560 (KR)**
• **KIM, Il Chul**
**Seoul 04560 (KR)**

(74) Representative: **J A Kemp LLP**
**80 Turnmill Street**
**London EC1M 5QU (GB)**

(54) **METHOD FOR SEPARATING DISODIUM 5'-INOSINATE**

(57) Provided is a method of separating 5'-inosinic acid from a microbial culture broth containing 5'-inosinic acid.

**EP 4 032 898 A1**

**Description**

**TECHNICAL FIELD**

The present disclosure relates to a method of separating disodium 5'-inosinate from a microbial culture.

**BACKGROUND ART**

Purine derivative nucleotides such as disodium inosinate (disodium 5'-inosinate, IMP2Na) are used as raw materials for preventive treatment of metabolic disorders, growth promoters, and anticancer drugs in the pharmaceutical sector, and are important substances used as food additives such as seasonings in the food sector. Disodium inosinate is manufactured through a fish meat extraction method of inosinic acid (IMP), a substance naturally present in meat, fish, etc., a RNA enzyme decomposition method, a microbial fermentation method, a combination of fermentation method and synthesis method, a combination of RNA chemical decomposition and synthesis, etc.

A lot of research has been conducted on the preparation method of inosinic acid, which is the precursor material for manufacturing disodium inosinate. In order to obtain disodium 5'-inosinate having such a quality that is suitable for use as a food additive, inosinic acid needs to be purified to remove impurities therefrom.

In relation to the method of culturing microorganisms and producing disodium 5'-inosinate from the culture thereof, an organic solvent can be used in the crystallization process. However, many organic solvents must be used, and accordingly, explosion-proof equipment and equipment for workers are required. In addition, in order to remove the organic solvent, a distillation process may be required and a corresponding equipment is required. Accordingly, there is a need for an alternative thereto.

**DESCRIPTION OF EMBODIMENTS**

**TECHNICAL PROBLEM**

The present disclosure provides a method of separating disodium 5'-inosinate, the method comprising: culturing 5'-inosinic acid-producing microorganisms; adjusting the pH of the culture broth of the microorganisms to be 7.4 to 8.0; concentrating the pH-adjusted culture broth to form disodium 5'-inosinate crystals; separating disodium 5'-inosinate crystals from the culture broth containing the disodium 5'-inosinate crystals; and washing the crystals by contacting the separated disodium 5'-inosinate crystals with a hydrophilic organic solvent.

**SOLUTION TO PROBLEM**

The term "5'-inosinic acid" is a nucleoside monophosphate, also called inosine monophosphate (IMP). "5'-inosinic acid" and "a salt thereof "are used interchangeably. The salt comprises "5'-inosinate" or disodium 5'-inosinate.

The term "culture" used herein refers to a resultant obtained by culturing a microorganism. The culture may comprise the microbial cells, or metabolites thereof. The metabolites may exist either intracellularly or extracellularly. The metabolites may be nucleic acid metabolites. The nucleic acid metabolites may be 5'-inosinic acid or a salt thereof.

The term "culture broth" used herein refers to a liquid portion obtained by removing microbial cells from a culture. The culture broth may comprise products excreted or derived from microorganisms. The culture broth may comprise soluble products. The culture broth may comprise 5'-inosinic acid or a salt thereof.

An aspect of the present disclosure provides a method of separating disodium 5'-inosinate, the method comprising: culturing 5'-inosinic acid-producing microorganisms; adjusting the pH of the culture broth of the microorganisms to be 7.4 to 8.0; concentrating the pH-adjusted culture broth to form disodium 5'-inosinate crystals; separating disodium 5'-inosinate crystals from the culture broth containing the disodium 5'-inosinate crystals; and washing the crystals by contacting the separated disodium 5'-inosinate crystals with a hydrophilic organic solvent.

The method may comprise obtaining a culture broth by removing a cell body from the microbial culture containing 5'-inosinic acid.

In the obtaining of the culture broth, the microorganism may be a microorganism producing 5'-inosine acid. The microorganism may be a genetically engineered microorganism to increase the production capacity of 5'-inosinic acid. The microorganisms may be bacteria. The microorganism may be a gram-positive or gram-negative bacterium. The bacterium may be a bacterium of the genus *Corynebacterium,* or the genus *Escherichia.* The bacterium may be *C. glutamicum, C. ammoniagenes,* or

*E. coli.* The microorganism may be to discharge 5'-inosinic acid to the outside of the cell.

The microbial culture may obtained by culturing the microorganism in the medium under conditions for producing 5'-inosinic acid. The microbial culture may comprise 5'-inosinic acid. The medium may be a known medium used for fermenting 5'-inosinic acid. The medium may comprise a carbon source, a nitrogen source, and trace elements. The medium may comprise molasses or a corn immersion solution. The medium may be a LB medium. The medium may comprise, per 1 L of distilled water, 46 g of glucose, 30 g of fructose, 10 g of yeast extract, 18 g of $KH_2PO_4$, 42 g of $K_2HPO_4$, 6 g of urea, 10 g of $MgSO_4*7H_2O$, 30 $\mu$g of biotin, and 5 mg of thiamine hydrochloride.

The microbial culture may comprise 5'-inosinic acid in an amount of 0.5 wt% to 20 wt%, 1.0 wt% to 20 wt%, 2.5 wt% to 20 wt%, 5 wt% to 20 wt%, or 5 wt% to 10 wt%, based on the total weight of the culture. The 5'-inosinic acid may be present in the extracellular medium. The microbial culture may have a pH of 6 to 9. The microbial culture may not have been subjected to ion exchange chromatography or crystallization using an organic solvent, for example, methanol.

Removal of a cell body is the removal of cells or cell debris from liquid components in culture. The removal of the cell body may be performed by a known method. The removal of the cell body may be performed by filtration, centrifugation, or a combination thereof. The method may comprise adjusting the pH of the culture broth to be from 7.4 to 8.0.

The adjusting the pH may be performed by adding an acid or a base to the culture broth. The acid may be a hydrochloric acid. The base may be NaOH. The pH may be a pH of 7.4 to 8.0, a pH of 7.6 to 8.0, a pH of 7.8 to 8.0, a pH of 7.4 to 7.8, or a pH of 7.4 to 7.6.

The method may comprise concentrating the pH-adjusted culture broth to form disodium 5'-inosinate crystals. The concentrating may be a crystallization process in which the culture broth is concentrated to convert 5'-inosinic acid into disodium 5'-inosinate crystals. This crystallization process is performed by simply increasing the concentration of 5'-inosinic acid in the culture broth by concentrating the culture broth, and does not use an organic solvent. The organic solvent not used therein may be a hydrophilic organic solvent. The hydrophilic organic solvent not used herein may be a C1-C5 alcohol, or a mixture of water and C1-C5 alcohol. The alcohol may be at least one selected from the group consisting of methanol, ethanol, propanol, isopropanol, and mixtures thereof. The hydrophilic organic solvent not used herein may be ethanol. The concentration of the ethanol may be from 50% to 70% in water based on the volume.

The concentrating of the pH-adjusted culture broth may be to reduce the amount of moisture in the culture broth. The concentrating may be to evaporate moisture by applying heat to the culture broth. The concentrating may be performed on the culture broth at a temperature of 50 °C or more, 60 °C or more, 70 °C or more, 80 °C or more, 90 °C or more, 50 °C to 90 °C, 50 °C to 80 °C, 50 °C to 70 °C, 50 °C to 60 °C, 60 °C to 90 °C, 60 °C to 80 °C, 60 °C to 70 °C, or 60 °C to 65 °C. The concentrating may be to perform concentration under reduced pressure. The concentrating may be performed until disodium 5'-inosinate crystals are formed by increasing the concentration of 5'-inosinic acid in the culture broth. The concentrating may be performed until the concentration of 5'-inosinic acid in the culture broth becomes 380 g/L to 600 g/L, 400 g/L to 550 g/L, 400 g/L to 500 g/L, 400 g/L to 475 g/L, or 425 g/L to 475 g/L. The concentrating may be performed until disodium 5'-inosinate crystals are formed and the culture broth has the shape of a slurry containing crystals. Hereinafter, a slurry solution containing crystals is also referred to as "crystal slurry." The term "slurry" used herein refers to a mixture of solids, for example, crystals, suspended in a liquid, usually water, with a specific gravity greater than 1. The crystals may have a particle size of 20 $\mu$m to 400 $\mu$m.

The method may further comprise concentrating the culture broth before the adjusting the pH of the culture broth. In this concentration, the disodium 5'-inosinate may not be formed in the culture broth, or may not be substantially formed. The concentrating may be to reduce the amount of moisture in the culture broth. The concentrating may be to evaporate moisture by applying heat to the culture broth. The concentrating may be performed until the concentration of 5'-inosinic acid in the culture broth becomes 150 g/L to 360 g/L. In the concentrating, the concentration of 5'-inosinic acid may be from 150 g/L to 360 g/L, 200 g/L to 300 g/L, 220 g/L to 280 g/L, or 240 g/L to 280 g/L. The concentrating may be performed on the culture broth at a temperature of 50 °C or more, 60 °C or more, 70 °C or more, 80 °C or more, 90 °C or more, 50 °C to 90 °C, 50 °C to 80 °C, 50 °C to 70 °C, 50 °C to 60 °C, 60 °C to 90 °C, 60 °C to 80 °C, 60 °C to 70 °C, or 60 °C to 65 °C.

The concentrating may be to perform concentration under reduced pressure. The method may further comprise cooling the culture broth including crystals formed after the concentrating. The cooling may be performed at a temperature of 25 °C to 45 °C, 25 °C to 40 °C, 25 °C to 35 °C, or 25 °C to 30 °C. The cooling may be performed by cooling the crystal slurry at a constant rate. The cooling may be performed at the cooling temperature for 1.0 hour to 3.0 hours, for example, 1.5 hours to 2.5 hours, or 2 hours. The constant cooling rate may be from 8.0 °C/hr to 17.0 °C/hr, for example, from 10.0 °C /hr to 15.0 °C/hr, from 1.0 °C/hr to 14.0°C/hr, from 2.0 °C/hr to 13.0 °C/hr, or about 12.5 °C /hr. The cooling may be performed while the formed crystal slurry is injected into a jacketed glass crystal tube and stirred. When the cooling is performed at a lower temperature, the solubility is lowered and more crystals may be recovered.

In addition, when the cooling is performed at a uniform rate, a harder and higher quality crystal may be obtained. The method may further comprise aging the crystals by incubating the slurry at the cooling temperature after cooling is complete. The incubation may be performed for 1 hour to 5 hours, 1 hour to 4 hours, 1 hour to 3 hours, 1.5 hours to 2.5 hours, or about 2 hours. The incubation may be performed while the crystal slurry is injected into a jacketed glass crystal tube and stirred.

The method may further comprise separating disodium 5'-inosinate crystals from the culture broth containing the formed disodium 5'-inosinate crystals.

The separating the crystal may be to perform a known crystal separation method within the scope not departing from the objectives of the present disclosure. The separating the crystals may be performed by, for example, centrifugation, filtration, or a combination thereof. The centrifugation may be performed at a rotation speed of 100 g to 1000 g, 100 g to 800 g, 100 g to 500 g, 300 g to 1000 g, or 500 g to 1000 g. The centrifugation may be performed for a rotation time of 10 minutes to 30 minutes.

The method may comprise washing the crystals by contacting the separated disodium 5'-inosinate crystals with a hydrophilic organic solvent.

In the washing process, the contacting may comprise mixing the crystals with a hydrophilic organic solvent. The washing may be to reduce the amount of impurities by removing impurities from the crystal. The hydrophilic organic solvent may be a C1-C5 alcohol or a mixture of water and a C1-C5 alcohol. The alcohol may be methanol, ethanol, propanol, isopropanol, butanol, pentanol, or a mixture thereof. The hydrophilic organic solvent may have a concentration of 50 % to 70 %, 60 % to 70%, 55.0 % to 65.0 %, or 60 % in water by volume. The hydrophilic organic solvent may be ethanol or a mixture of water and ethanol. The ethanol may be advantageous in that it is inexpensive and has low toxicity. The hydrophilic organic solvent may be, based on volume, 10 % to 50 %, for example, 20 % to 50 %, 15 % to 40 %, 20 % to 40 %, 25 % to 40 %, 25 % to 35 %, or 25 % to 50 %, of the formed crystal-containing sample.

In the method, the separating the crystal and the washing the crystal may be performed simultaneously. In addition, the separating the crystal and the washing the crystal may be performed separately.

The method of the present disclosure may further comprise removing a coloring material by contacting the washed crystals with activated carbon in an aqueous solvent. The contacting may be mixing the washed crystals with activated carbon in an aqueous solvent and incubating the same. The aqueous solvent may be water.

The removing the coloring material may comprise dissolving the washed crystals in water to a concentration of 250 g/L to 500 g/L and adding, to an aqueous solution containing crystals, 0.1 % to 10 % of activated carbon based on the weight of 5'-inosinic acid. The contacting may be performed at 40 °C to 80 °C.

## EFFECTS OF DISCLOSURE

According to the method of separating disodium 5'-inosinate according to the present disclosure, disodium 5'-inosinate crystals can be efficiently separated. In particular, according to the method, since an organic solvent is used only in the washing process, the amount of the organic solvent is small. According to the method, the separated disodium 5'-inosinate crystals have a high separation yield and high purity.

In detail, by adjusting the pH of the culture broth, it is possible to significantly increase the separation yield of disodium 5'-inosinate crystals. In addition, by contacting the separated disodium 5'-inosinate crystals with a hydrophilic organic solvent to wash the crystals, the decrease in the yield may be prevented compared to the case of washing with water, and the color of the formed crystals is improved, and the amount of harmful organic solvents in the crystal is significantly reduced. Accordingly, economic feasibility can be secured.

## MODE OF DISCLOSURE

Hereinafter, the present disclosure will be described in more detail through examples. However, these examples are for illustrative purposes only, and the scope of the present disclosure is not limited to these examples.

**Example: IMP Fermentation and IMP Isolation from Culture**

**1. IMP production and culture of microorganisms**

*Corynebacterium glutamicum* was inoculated in a 30 L fermentation incubator containing 18 L of medium and cultured at 31 °C for 140 hours to generate 5'-inosinic acid (IMP) in the culture. The 5'-inosinic acid was secreted out of the cell and was present in the culture broth. The medium had an initial pH of 7.2 and contained, per 1 L of distilled water, 46 g of glucose, 30 g of fructose, 10 g of yeast extract, 18 g of $KH_2PO_4$, 42 g of $K_2HPO_4$, 6 g of urea, 10 g of $MgSO_4*7H_2O$, 30 $\mu$g of biotin, and 5 mg of thiamine hydrochloride. The pH of the final culture was 6.8.

After adjusting the pH to 10.5 by adding NaOH of 50 w/w% to the resulting culture, the cells were removed by filtration through a microfilter, and a supernatant was obtained. The concentration of 5'-IMP in the supernatant was measured using HPLC. The supernatant contained 140 g/L of 5'-IMP.

**2. Formation of disodium 5'-inosinate crystals**

2 L of the filtrate was put into a rotary evaporator (N-1110V: EYELA, Japan), and moisture in the filtrate was evaporated, and the filtrate was concentrated until the concentration of 5'-IMP reached 260 g/L. Evaporation was performed using a vacuum controller NVC-2200 at an evaporator internal pressure of 120 mmHg, a vessel temperature of 65 °C, and an evaporation rate of 1 L/hr. At this time, the temperature of the filtrate inside the vessel was 55 °C. This concentration process is hereinafter referred to as "primary concentration."

The resulting concentrated filtrate had a pH of 9.0 and a temperature of 55 °C. The change in the pH of the initial filtrate from 10.5 to 9.0 appears to be due to the removal of ammonia during the evaporation process.

Next, with respect to 1077 mL of the obtained concentrated filtrate, 4.5 mL of 35% (v/v) hydrochloric acid per 1 L of the obtained concentrated filtrate was added to adjust the pH to 7.4, and the temperature was maintained at 55 °C. Hereinafter, this process is referred to as "pH adjustment process".

1082 mL of the concentrated filtrate, whose pH had been adjusted, was placed in the rotary evaporator, and under the same conditions as described above, the concentrated filtrate was concentrated until a supersaturated solution in which 460 g/L of 5'-IMP crystals and liquids in the filtrate were mixed, that is, a slurry state was obtained. Here, the concentration "460 g/L" of 5'-IMP means the amount of both 5'-IMP and 5'-IMP2Na crystals dissolved. As a result, 5'-IMP and 2Na$^+$ ions in 608 mL of the supersaturated solution were precipitated as disodium 5'-inosinate (hereinafter also referred to as "5'-IMP2Na") crystals, and thus the concentrated filtrate became a viscous suspension in which solid crystals were mixed with the liquid filtrate (hereinafter also referred to as "slurry"). Immediately after the crystallization was finished, the temperature of the obtained slurry was 55 °C and the pH thereof was 8.0. This process is hereinafter referred to as "secondary concentration and crystallization process." That is, since 5'-IMP2Na crystals were formed by the secondary concentration and the crystallization process, this indicates that 5'-IMP can be converted into 5'-IMP2Na crystals by concentration without using an organic solvent. This is a significant effect that is unexpected for a person skilled in the art.

608 mL of the slurry was poured into a jacketed glass crystal tube, and an overhead stirrer (eyela, zz-2121) was installed on the top thereof and the slurry was cooled at a cooling rate of 12.5 °C/hr to 30 °C for 2 hours while stirring at 200 rpm. The jacketed glass crystal tube was used to cool the slurry at a constant rate. After cooling was finished, incubation was performed at 30 °C for 2 hours while stirring the jacketed glass crystal tube containing the slurry. This process corresponds to the aging process to allow the crystal growth to continue. Hereinafter, this process is referred to as "cooling and aging process".

**3. Recovery of disodium 5'-inosinate crystals**

5'-IMP2Na crystals were isolated from the obtained aged slurry. In detail, 608 mL of the obtained aged slurry was placed in a basket separator H-110F (KOKUSAN Co. Ltd., Japan) and centrifuged at 340xg for 20 minutes. The H-110F centrifuge has a perforated basket installed therein, and the basket is connected to an external rotation supply. The perforated basket was made of polyamide multifilament fiber filter fabric, and the air transmittance of the filter was 250 L/m$^2$/s at 2 mbar. As a result of the centrifugation, 300 g of a cake containing IMP2Na crystals, which was prepared by removing the liquid from the slurry, was obtained.

Next, 150 ml of a 60% (v/v) aqueous ethanol solution was sprayed thereto to wash IMP2Na crystals (hereinafter also referred to as "ethanol washing"). As a result of washing, 272 g of 5'-IMP2Na crystals were recovered, and then dried at room temperature for 24 hours to obtain 265 g of dried columnar 5'-IMP2Na crystals. This 5'-IMP2Na crystal is also called 5'-IMP2Na crystal 7.5 hydrate.

Purity and transmittance of the obtained dried 5'-IMP2Na crystal were measured. As a result, the dried 5'-IMP2Na crystal had a yield of 94.6%, a purity of 95.7%, and a transmittance of 86.10%. The yield and purity were calculated according to the following formula.

[Equation 1]

$$\text{Yield} = \text{Weight of the obtained 5'-IMP2Na crystal/Weight of 5'-IMP2Na crystal in 2L of the filtrate} \times 100$$

In order to determine the purity, 1.0 g of the dried 5'-IMP2Na crystal and 1.0 g of standard 5'-IMP2Na crystal (Sigma, ≥9.0% (HPLC)) were each dissolved in 1 L of tertiary distilled water to prepare 1.0 g/L concentration of experimental group and 1.0 g/L concentration of standard product solution, respectively. 5 μL of experimental solution and 5 μL of standard solutions were loaded into the column in an Agilent 1260 Infinity Quaternary LC (Agilent Technology Inc.) system. The column was a Shiseido CAPCELL PAK C18 ACR (150 mm x 4.6 mm, 3 μm). Next, the absorbance at 254 nm of the eluate flowing out while flowing acetonitrile 2% (v/v)/phosphate buffer (pH 2.4) 98% (v/v) to the column at a flow rate of 1 ml/min, was measured. The phosphate buffer contained 2 g/L of ammonium phosphate, 0.2 g/L of tetrabutyl ammonium phosphate, and 0.82 g/L of phosphoric acid. At this time, the temperature was 35 °C. This HPLC condition was also used to measure the concentration of 5'-IMP in the filtrate. As a result, the purity was calculated according to the following formula.

[Equation 2]

$$\text{Purity} = 5'\text{-IMP weight/weight of solids} \times 100$$

In addition, a solution obtained by dissolving the experimental group in water at a concentration of 5 (w/v)% for transmittance measurement, was placed in a rectangular cell of the CARY 100 UV-VIS (Agilent Technology Inc.) instrument, and the transmittance thereof was measured at 420 nm.

As the experiment for Control 1, 5'-IMP2Na crystals were recovered in the same manner as described above except that, in the "recovery of disodium 5'-inosinate crystals", 60% (v/v) aqueous ethanol solution was not used in the washing process (hereinafter also referred to as "no washing"). As a result, the obtained purified crystal of 5'-IMP2Na had a dry weight of 266 g, a yield of 95%, a purity of 86.40%, and a transmittance of 40.48%.

As the experiment for Control 2, 5'-IMP2Na crystals were recovered in the same manner as described above except that, in the "recovery of disodium 5'-inosinate crystals", deionized water in an amount of 25% relative to the volume of the 5'-IMP2Na crystal slurry was used instead of a 60% (v/v) aqueous ethanol solution (hereinafter also referred to as "water washing"). As a result, the obtained purified crystal of 5'-IMP2Na had a dry weight of 240 g, a yield of 85.7%, a purity of 94.64%, and a transmittance of 72.72%.

[Table 1]

|  | Dry weight (g) | Yield (%) | Purity (%) | Transmittance (%) |
|---|---|---|---|---|
| Experimental group | 265 | 94.6 | 95.70 | 86.10 |
| Control 1 | 266 | 95.0 | 86.40 | 40.48 |
| Control 2 | 240 | 85.7 | 94.64 | 72.72 |

Table 1 shows the dry weight, yield, purity, and transmittance of 5'-IMP2Na crystals obtained in the experimental group, Control 1, and Control 2. As shown in Table 1, when a 60% (v/v) aqueous ethanol solution was used in the crystal separation process, the purity and transmittance of the crystals were improved at the same time compared to the case where no washing was performed or the case of washing with water. In detail, in the case of the experimental group, purity was increased by 10.8% and 1.1%, and transmittance was increased by 112.7% and 18.4%, compared to Control 1 and Control 2, respectively. This increase in purity and transmittance is a significant effect unexpected from the prior art. In particular, the fact that the transmittance was increased by 112.7% and 18.4% indicates that the produced 5'-IMP2Na crystal could easily satisfy food standards. In detail, in order to meet the standards for food, the transmittance needs to be at a level of 98%, and to obtain this level of transmittance, Control 1 and Control 2 need to perform recrystallization two or more times, whereas the experimental group can achieve the standard for food even by performing one additional recrystallization. In addition, in order to adjust Control 1 and Control 2 to be the level of the experimental group, an increase in the amount of washing is required, and the recovery rate may be decreased.

**4. Influence of pH in the crystallization process**

In this section, the effect of the pH of the solution on the crystallization of 5'-IMP in the crystallization process was confirmed.

In detail, the pH to 7.2 was adjusted by adding 35% (v/v) hydrochloric acid to 2000 mL of the concentrated filtrate (pH 9.0) obtained after "primary concentration" in "2. disodium 5'-inosinate crystal formation" and the temperature was maintained at 55 °C.

2000 mL of the concentrated filtrate, whose pH had been adjusted, was placed in the rotary evaporator, and under the same conditions as described above, the concentrated filtrate was concentrated until a supersaturated solution in which 400 g/L of 5'-IMP crystals and liquids in the filtrate were mixed, that is, a slurry state was obtained. Here, the concentration "400 g/L" of 5'-IMP means the amount of both 5'-IMP and 5'-IMP2Na crystals dissolved. As a result, 5'-IMP and 2Na$^+$ ions in 608 mL of the supersaturated solution were precipitated as 5'-IMP2Na crystals, and the concentrated filtrate became a viscous suspension in which solid crystals and liquid filtrate were mixed (hereinafter " also referred to as "slurry"). Immediately after the crystallization was finished, the temperature of the obtained slurry was 55 °C and the pH thereof was 8.0.

608 mL of the slurry was poured into a jacketed glass crystal tube, and an overhead stirrer (eyela, zz-2121) was installed on the top thereof and the slurry was cooled at a cooling rate of 12.5 °C/hr to 30 °C for 2 hours while stirring at 200 rpm. The jacketed glass crystal tube was used to cool the slurry at a constant rate. After cooling was finished, incubation was performed at 30 °C for 2 hours while stirring the jacketed glass crystal tube containing the slurry. This process corresponds to the aging process to allow the crystal growth to continue. Hereinafter, this process is referred to as "cooling and aging process".

The pH of 608 mL of the cooled filtrate was adjusted to be 7.4 to 9.0 by using 35 %(v/v) hydrochloric acid and 50% NaOH solution. The supernatant of the filtrate at each pH was loaded into HPLC as described above and eluted to measure the concentration of 5'-IMP in the filtrate. Table 2 shows the concentration of 5'-IMP in the filtrate according to pH.

[Table 2]

| pH | 7.2 | 7.4 | 7.6 | 7.8 | 8.0 | 8.2 | 8.4 | 8.6 | 8.8 | 9.0 |
|---|---|---|---|---|---|---|---|---|---|---|
| IMP concentratio n (g/L) | 60.2 | 52.7 | 48.3 | 49.0 | 52.4 | 55.4 | 60.6 | 73. 7 | 86.0 | 113. 7 |

As shown in Table 2, the concentration of 5'-IMP was the lowest at the pH of 7.4 to 8.0. In Table 2, the low solubility at the pH of 7.4 to 8.0 means that 5'-IMP2Na crystals are well formed in this pH range.

### 5. Influence of organic solvent, concentration and amount used in the separation of disodium 5'-inosinate crystals

In this section, regarding the "3. Recovery of disodium 5'-inosinate ", the effects of other organic solvents, instead of 60% (v/v) ethanol aqueous solution, concentration, and amounts on the solubility of the 5'-IMP, the color of 5'-IMP2Na crystls, and the purity of the 5'-IMP2Na crystals, the amount of moisture and the absorbance were confirmed.

(1) Solubility of 5'-IMP depending on organic solvents and absorbance of solutions in which crystals were dissolved

608 ml of the aged slurry obtained in "2. disodium 5'-inosinate crystal formation" was put into 1000 ml of the organic solvent shown in Table 3 and stirred at 25 °C for 1 hour to allow 5'-IMP in the slurry to be dissolved in the organic solvent. This solution was centrifuged at 1500 rpm for 10 minutes to obtain a supernatant.

Thereafter, the concentration of 5'-IMP in the supernatant was measured using HPLC. The crystals were separated as described in "3. Recovery of disodium 5'-inosinate crystals" and dissolved in water to make a 5 (w/v)% aqueous solution, and absorbance thereof at 420 nm was measured. Since the main impurities in the crystal were yellow-brown, the absorbance of 420 nm was selected to measure the amount of these color substances. Absorbance was measured using a CARY 100 UV-VIS (Agilent Technology Inc.) instrument.

Table 3 shows the solubility of 5'-IMP according to the type and concentration of the organic solvent. In Tables 3 and 4, % is based on a volume/volume basis.

[Table 3]

| Class | 10% | 30% | 50% | 60% | 70% | 90% | 100% |
|---|---|---|---|---|---|---|---|
| Water | - | - | - | - | - | - | 178.86 |
| Methanol | 100.71 | 29.76 | 4.91 | 2.51 | 1.50 | 0.80 | 1.14 |
| Ethanol | 93.32 | 12.59 | 1.97 | 1.23 | 0.12 | 0.00 | 0.93 |
| Isopropanol | 96.02 | 15.63 | 2.39 | 1.76 | 0.11 | 0.00 | 0.00 |

Table 4 shows the absorbance of a solution obtained by dissolving 5'-IMP2Na crystals, obtained by using different types and concentrations of organic solvents, in water.

[Table 4]

| Class | 10% | 30% | 50% | 60% | 70% | 90% | 100% |
|---|---|---|---|---|---|---|---|
| Water | - | - | - | - | - | - | 0.03 |
| Methanol | 0.03 | 0.04 | 0.04 | 0.05 | 0.06 | 0.14 | 0.23 |
| Ethanol | 0.04 | 0.04 | 0.04 | 0.08 | 0.12 | 0.35 | 0.44 |
| Isopropanol | 0.03 | 0.03 | 0.04 | 0.17 | 0.22 | 0.44 | 0.50 |

As shown in Tables 3 and 4, when the concentration of the hydrophilic organic solvent was 50% to 70%, it was confirmed that the concentration of 5'-IMP in the mother solution was low and the absorbance of the washed crystals was low.

(2) Characteristics of crystals according to amount of organic solvent

5'-IMP2Na crystals were separated, and crystal purity, crystal moisture and absorbance were measured in the same manner as described above, except that the amount of 60% (v/v) ethanol aqueous solution used in "3. Recovery of disodium 5'-inosinate crystals" was changed from 0 %(v/v) to 40 %(v/v) relative to the volume of the slurry. Crystalline moisture was measured by the dry oven method, in which the weight was measured before and after drying at 125 °C for 3 hours in an oven. Crystal purity, and absorbance were determined as described above.

[Table 5]

| Class | 0% | 5% | 15% | 25% | 35% | 40% |
|---|---|---|---|---|---|---|
| Crystalline purity (%) | 86.4 | 91.18 | 92.60 | 95.7 | 96.00 | 96.01 |
| Crystal Moisture (%) | 29.46 | 30.40 | 30.89 | 30.83 | 30.62 | 30.56 |
| Absorbance | 0.42 | 0.24 | 0.17 | 0.12 | 0.09 | 0.09 |

As shown in Table 5, in the case of a 60% (v/v) aqueous ethanol solution, which is a washing solution, when the amount thereof was 25% or more, for example, 25% to 35% of the volume of the crystal slurry, crystal purity, crystal moisture and absorbance were simultaneously improved.

**Claims**

1. A method of separating disodium 5'-inosinate, the method comprising: culturing a microorganism that produces 5'-inosinic acid;

   adjusting the pH of a culture broth of the microorganism to be from 7.4 to 8.0; concentrating the pH-adjusted culture broth to form disodium 5'-inosinate crystals; separating disodium 5'-inosinate crystals from the culture broth containing the disodium 5'-inosinate crystals; and
   washing the crystals by contacting the disodium 5'-inosinate crystals with a hydrophilic organic solvent.

2. The method of claim 1, wherein the culture broth of the microorganism comprises 5 wt% to 20 wt% of 5'-inosinic acid based on the total weight of the culture broth.

3. The method of claim 1, wherein the microorganism is a bacterium of the genus *Corynebacterium* or genus *Escherichia*.

4. The method of claim 1, wherein the concentrating is to perform concentrating under reduced pressure.

5. The method of claim 1, wherein the concentrating is performed until the concentration of 5'-inosinic acid in the culture broth becomes 400 g/L to 550 g/L.

6. The method of claim 1, further comprising concentrating the culture broth before the adjusting the pH.

7. The method of claim 6, wherein the concentrating the culture broth before the adjusting the pH is performed until the concentration of 5'-inosine acid in the culture broth becomes 150 g/L to 360 g/L.

8. The method of claim 6, wherein the concentrating the culture broth before the adjusting the pH is to perform concentrating under reduced pressure.

9. The method of claim 1, comprising cooling formed crystals after the concentrating.

10. The method of claim 9, wherein the cooling is performed at a temperature of 25 °C to 30 °C.

11. The method of claim 1, wherein the hydrophilic organic solvent is a C1-C5 alcohol.

12. The method of claim 11, wherein the alcohol is at least one selected from the group consisting of methanol, ethanol, propanol, and isopropanol.

13. The method of claim 1, wherein the hydrophilic organic solvent is ethanol.

14. The method of claim 1, wherein the concentration of the hydrophilic organic solvent is 50 %(v/v) to 70 %(v/v).

15. The method of claim 1, wherein the volume of the hydrophilic organic solvent is 20 % to 50 % of the total volume of a sample including formed crystals.

16. The method of claim 1, further comprising contacting washed crystals with activated carbon in an aqueous solvent to remove a coloring material.

<table>
<tr><td colspan="2" align="center">**INTERNATIONAL SEARCH REPORT**</td><td>International application No.<br><br>**PCT/KR2020/012364**</td></tr>
</table>

**A.   CLASSIFICATION OF SUBJECT MATTER**

**C07H 19/20**(2006.01)i; **C07H 1/08**(2006.01)i

According to International Patent Classification (IPC) or to both national classification and IPC

**B.   FIELDS SEARCHED**

Minimum documentation searched (classification system followed by classification symbols)

C07H 19/20(2006.01); A23L 27/22(2016.01); C08G 63/183(2006.01); C12N 1/20(2006.01); C12P 1/02(2006.01); C12P 19/26(2006.01); C12P 19/28(2006.01); C12P 19/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 5'-이노신산(5'-inosinic acid), Corynebacterium, Escherichia, 배양(culture), 농축(concentration), 유기용매(organic solvent)

**C.   DOCUMENTS CONSIDERED TO BE RELEVANT**

| Category* | Citation of document, with indication, where appropriate, of the relevant passages | Relevant to claim No. |
|---|---|---|
| A | KR 10-1978-0000599 B1 (CHEIL JEDANG CORPORATION) 28 November 1978 (1978-11-28)<br>    See page 2. | 1-16 |
| A | KR 10-2009-0072584 A (CJ CHEILJEDANG CORPORATION) 02 July 2009 (2009-07-02)<br>    See abstract; and claims 1-9. | 1-16 |
| A | KR 10-2008-0007985 A (CJ CORP) 23 January 2008 (2008-01-23)<br>    See abstract; and claims 1-11. | 1-16 |
| A | US 5164306 A (YOSHIHARA, Y. et al.) 17 November 1992 (1992-11-17)<br>    See abstract; and claims 1-6. | 1-16 |
| A | KR 10-1997-0007935 B1 (MIWON CO.) 19 May 1997 (1997-05-19)<br>    See abstract; and claims 1-4. | 1-16 |

☐ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 January 2021** | **07 January 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

**PCT/KR2020/012364**

| Patent document cited in search report | | | Publication date (day/month/year) | Patent family member(s) | | | Publication date (day/month/year) |
|---|---|---|---|---|---|---|---|
| KR | 10-1978-0000599 | B1 | 28 November 1978 | None | | | |
| KR | 10-2009-0072584 | A | 02 July 2009 | CN | 101918576 | A | 15 December 2010 |
| | | | | CN | 101918576 | B | 26 March 2014 |
| | | | | KR | 10-0926253 | B1 | 12 November 2009 |
| | | | | WO | 2009-084836 | A2 | 09 July 2009 |
| | | | | WO | 2009-084836 | A3 | 11 September 2009 |
| KR | 10-2008-0007985 | A | 23 January 2008 | CN | 101109017 | A | 23 January 2008 |
| | | | | KR | 10-0828706 | B1 | 09 May 2008 |
| US | 5164306 | A | 17 November 1992 | None | | | |
| KR | 10-1997-0007935 | B1 | 19 May 1997 | KR | 10-1996-0003603 | A | 23 February 1996 |

Form PCT/ISA/210 (patent family annex) (July 2019)